(19) Europäisches Patentamt
European Patent Office
Office européen des brevets

(11) Publication number: **0 378 881 B1**

(12) # EUROPEAN PATENT SPECIFICATION

(45) Date of publication of patent specification: **09.06.93**

(51) Int. Cl.$^5$: **C07K 7/00**, C07K 7/08, A61K 37/02, A61K 39/00

(21) Application number: **89203318.4**

(22) Date of filing: **27.12.89**

(54) **Synthetic peptides and their use as universal carriers for the preparation of immunogenic conjugates suitable for the development of synthetic vaccines.**

(30) Priority: **17.01.89 IT 1911089**
**16.11.89 IT 2240989**

(43) Date of publication of application:
**25.07.90 Bulletin 90/30**

(45) Publication of the grant of the patent:
**09.06.93 Bulletin 93/23**

(84) Designated Contracting States:
**AT BE CH DE ES FR GB GR LI LU NL SE**

(56) References cited:
**EP-A- 0 166 410**
**EP-A- 0 209 643**
**EP-A- 0 289 110**
**EP-A- 0 320 034**
**WO-A-86/05790**
**GB-A- 2 193 215**
**GB-A- 2 199 038**

**NATURE, vol. 328, 16th July 1987, pages 257-259; D.A. HERRINGTON et al.: "Safety and immunogenicity in man of a synthetic peptide malaria vaccine against Plasmodium falciparum sporozoites"**

(73) Proprietor: **ENIRICERCHE S.p.A.**
**Corso Venezia 16**
**I-20121 Milan(IT)**

(72) Inventor: **Pessi, Antonello**
**Via Massaciuccoli 19**
**I-00199 Rome(IT)**
Inventor: **Bianchi, Elisabetta**
**Via Sabotino 17**
**I-00195 Rome(IT)**
Inventor: **Verdini, Antonio Silvio**
**Via S. Martino 12**
**I-00015 Monterotondo Rome(IT)**
Inventor: **Corradin, Giampietro**
**Prad-dom-nicodiz**
**CH-1000 Losanna 26(CH)**

(74) Representative: **Roggero, Sergio et al**
**Ing. Barzanò & Zanardo Milano S.p.A. Via Borgonuovo 10**
**I-20121 Milano (IT)**

**EP 0 378 881 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

CHEMICAL ABSTRACTS, vol. 110, 1989, page 513, abstract no. 112726n, Columbus, Ohio, US; J.M. CLAVERIE et al.: "T-immunogenic peptides are constituted of rare sequence patterns. Use in the identification of T epitopes in the human immunodeficiency virus gag protein", & EUR. J. IMMUNOL. 1988, 18(10), 1547-53

CHEMICAL ABSTRACTS, vol. 110, 1989, page 540, abstract no. 133241r, Columbus, Ohio, US; L.F. SMITH et al.: "The role of lauroyl and Cys-Tyr-Gly-Gly in T cell activation by synthetic peptides from Plasmodium falciparum circumsporozoite protein", & UCLA SYMP. MOL. CELL. BIOL., NEW SER. 1988, 84 (TECHNOL. ADV. VACCINE DEV.), 651-9

CHEMICAL ABSTRACTS, vol. 107, 1987, pages 529-530, abstract no. 173783p, Columbus, Ohio, US; M.R. HOLLINGDALE et al.: "Plasmodium falciparum: elicitation by peptides and recombinant circumsporozoite proteins of circulating mouse antibodies inhibiting sporozoite invasion of hepatome cells", & EXP. PARASITOL. 1987, 63(3), 345-51

CHEMICAL ABSTRACTS, vol. 110, 1989, page 514, abstract no. 112732m, Columbus, Ohio, US; S. DEMOTZ et al.: "Delineation of several DR-restricted tetanus toxin T cell epitopes", & J. IMMUNOL. 1989, 142(2), 394-402

NATURE, vol. 332, 10th March 1988, pages 158-161; M.E. PATARROYO et al.: "A synthetic vaccine protects humans against challenge with asexual blood stages of Plasmodium falciparum malaria"

CHEMICAL ABSTRACTS, vol. 110, 1989, abstract no. 82471f, Columbus, Ohio, US; relates to US PAT. APPLIC. US 19000 (DEPARTMENT OF HEALTH AND HUMAN SERVICES)

## Description

The present invention relates to synthetic peptides to their use as universal carriers for the preparation of immunogenic conjugates and to the use of said conjugates in the development of synthetic vaccines able to induce protective immunity against different pathogenic agents which is not genetically restricted or only slightly so.

The term "hapten" signifies a molecule able to bind to specific antibodies (antigenic) but not to induce their formation or to induce it to only a low antibody count (not immunogenic).

Specifically, haptens include peptide or polysaccharide molecules (thymo-independent antigens) isolated and purified from pathogenic agents or short peptides with a sequence corresponding to that of one or more B epitopes of a natural antigen obtained by chemical synthesis or via recombinant DNA.

The term "antigen" signifies a molecule able to induce the formation of specific antibodies (immunogenic) and to react with them (antigenic).

Antigens include natural microorganisms, cells and their soluble products.

Immunisation against infection caused by pathogenic agents (viruses, parasites and bacteria) is generally obtained by inoculating an individual with a vaccine containing a natural antigen (i) or antigens (attenuated or killed microorganisms or their suitably treated products) which stimulates the production of antibodies able to neutralise the antigen itself. The use of said traditional vaccines has however numerous drawbacks deriving both from the limited availability of the natural antigenic material and from the danger of possible infection while handling said pathogenic material.

In addition, these vaccines require preparation and storage (low temperature) conditions which can constitute an enormous problem, especially in underdeveloped countries.

For all these reasons there is currently an increasing interest in the development of synthetic vaccines which contain not the whole pathogen or its products but instead short peptide fragments which reproduce segments of the natural antigen, which are known as epitopes or determinants or antigenic sites.

The preparation of such a vaccine therefore requires the identification and the selection of the peptides to be used.

It is known that an immune response towards a foreign agent by an organism involves the cooperation of various types of cells, namely antigen-presenting cells (APC), B lymphocytes (antibody producers able to function as APC), T-helper (or Th) lymphocytes and T-cytotoxic lymphocytes responsible for directly killing the cells infected by the pathogen.

The immunity can be humoral (mediated by the antibodies produced by the B cells) or can be cell-mediated.

Considering humoral immunity, the minimum requirement for initiating an effective immune response is that the B lymphocytes circulating within the organism of an antigenic determinant recognise the foreign substance (B epitope) and that the Th cells of a determinant, generally different from B, recognise the substance (T epitope).

Consequently two peptide sequences must be present in the synthetic vaccine as a minimum requirement for its effectiveness, namely the B epitope and the T epitope.

While the identification of the B epitope or epitopes contained in a natural antigen is facilitated by the possibility of using natural anti-pathogenic antibodies as reactants, localizing the T epitopes presents very complicated problems.

In this respect, the first case involves direct interaction between the anti-pathogenic antibody and the antigen, whereas in the second case the activation of the T cell clone specific for an epitope of the antigen takes place only after this has been interiorized by an APC, processed by proteolysis or denaturation or both in short peptide fragments, and then re-exteriorized on the surface of the APC in association with a membrane molecule of the class II cell (for the T-helpers) encoded by a gene of the major histocompatibility complex (MHC). The Th lymphocyte receptor therefore recognises not the free antigen but the complex formed between its fragment and a part of the class II MHC molecule.

These molecules are grouped in three families DR, DP and DQ for man (HLA) and IA and IE for the mouse, and are extremely polymorphous.

The polymorphism is due to a large number of alleles for each of the encoding genes for this molecule.

This diversity allows a large number of combinations in a chromosome (haplotype).

It is therefore very difficult to find individuals not linked by family relationship who have an identical MHC.

The fact that only some of the peptides deriving from the antigen degradation are able to associate with a host-histocompatibile antigen means that the gene set of the host, which encodes said antigens, restricts the number of peptides able to form a complex with them, this however being an essential condition for

activation of the Th lymphocytes.

The term "genetic restriction" of the T epitopes is therefore used in the sense that said peptide fragments are restricted in their interaction with the T lymphocyte receptor by the proteins with which they are associated.

This is the basis of the difficulty of identifying within a natural antigen those T epitope or epitopes with absent or minimum genetic restriction and suitable for the development of synthetic acellular vaccines with wide effectiveness, ie able to induce protective immunization against the pathogenic agent of interest in individuals with different MHC gene sets.

Up to the present time this difficulty has been very often overcome by using as the source of T epitopes a macromolecule, also known as a carrier, to which a natural or synthetic peptide or polysaccharide hapten derived from a pathogenic agent of interest is covalently bound. Examples of carriers suitable for this purpose are the tetanus toxoid (TT), the diphtheria toxoid (CRM), serum albumin and lamprey hemocyanin (KLH) in that they provide the resultant conjugate with minimum genetic restriction. Conjugates comprising said carriers, known also as universal carriers, are able to function as T cell clone activators in individuals having very different gene sets.

In this respect, most of the animals used for in vivo tests and having different gene sets are able to activate an antibody response towards the conjugate antigen, ie they are responders. Even though this approach to the problem has proved effective, the use of macromolecular-hapten carrier conjugates in a process of immunization against a pathogenic agent still has numerous drawbacks due to the difficulty of standardizing their various preparation stages, the possible alteration of the antigenic properties of the hapten as a result of the conjugation reaction [J.P. Briand et al., J. Immunol. Methods, 78 (1985) 59-69],and finally the phenomenon known as "epitope suppression induced by the carrier" which causes suppression of anti-hapten antibody production in an individual already immunized with only the carrier [H. Etlinger et al., J. Immunol. 140, (1988) 626]. This phenomenon is observed in those cases in which the carrier used is a protein to which the host has already been exposed, such as the tetanus toxoid used for anti-tetanus vaccination.

In addition, the use of short peptide sequences as immunogens, even if these comprise both a B epitope and a T epitope of the natural antigen, has generally led to an immune response which is genetically much more restricted, ie a reduction in the number of responder animals and/or individuals [see for example AR. Togna et al., J. Immunol., 137 (1986) 2956-2960; G. Del Giudice et al., J. Immunol. 137, (1986), 2962-2955; G. Del Giudice et al., Immunology 63, (1988) 187-191].

Recently Fairweather N.F. et al have reported in SF 209281 published on 21.1.87 the cloning of the tetanus toxin gene and its characterisation in terms of the nucleotide and amino acid sequence of the B and C fragments of said protein.

This European patent application does not however either teach or suggest the T epitope sequence of said protein.

U. Eisel et al. [EMBO J. 5 (1986), 2495-2502] describe the complete sequence of the tetanus toxin and report the localization of the promotor of the gene of said toxin.

Eisel et al. neither teach nor suggest the sequence of the T epitopes contained in said protein. Again, Fairweather et al. [Infect. Imm., 55, (1987) 2541-2545) teach the preparation via recombinant DNA of fragments of the tetanus toxin with high molecular weight and their use in the immunization of mice against tetanus. Said work does not report either the localization of the T epitopes or their sequence. The object of the present invention is to provide universal peptide carriers for use in the preparation of immunogenic peptide-hapten conjugates suitable for developing synthetic vaccines with very wide effectiveness and protective against different pathogenic agents.

This object is attained according to the present invention by localizing within the tetanus toxin, T epitopes able to be recognised by numerous human T cell clones within the context of a wide range of alleles of the human major histocompatibility complex made by synthesizing peptides having the amino acid sequence corresponding to that of said T epitopes and of their analogs.

Thus the present invention firstly provides synthetic peptides having an amino acid sequence corresponding to that of those tetanus toxin T epitopes and their analogs able to be recognised by numerous human Th cell clones within the context of a wide range of alleles of the human major histocompatibility complex.

The present invention still provides the use of said synthetic peptides as universal carriers in the preparation of immunogenic conjugates.

The present invention further provides immunogenic conjugates consisting of said synthetic peptides covalently bound to a natural or synthetic peptide or polysaccharide hapten derived from a pathogenic agent of interest.

The present invention also relates to the use of said immunogenic conjugates in the preparation of synthetic vaccines having wide effectiveness and protective against pathogenic agents of interest.

The present invention also provides synthetic vaccines for immunizing individuals with different MHC gene sets against infections caused by a pathogenic agent, which contain an immunologically effective quantity of said immunogenic conjugates.

Further objects of the present invention will be apparent from reading the text and the following examples.

According to the present invention, the antigenic T sites of the tetanus toxoid (TT) were localized using T cell clones specific for TT which were isolated from the peripheral blood of a donor with a DR 3,5 gene set who had been immunized with tetanus toxoid as described by Lanzavecchia, A., Nature 314, 537, (1985).

The clones were kept under culture by periodical restimulation using irradiated peripheral blood mononucleate cells (PBMC) which were autologous, ie derived from the same donor, plus 1% of phytohemagglutinin.

After 5 days the T blast-cells were washed and cultivated in RPMI-c culture medium (RPMI 1640, GIBCO Laboratories, Paisley, Scotland) supplemented with 10% of unactivated calf foetal serum, 2mM L-glutamine, 1 mM sodium pyruvate, 5 x $10^{-5}$ M 2-mercaptoethanol and 1% of a mixture 100 x of non-essential amino acids (GIBCO) to which 40 units/ml of recombinant human interleukin 2 (Hoffmann-La Roche) were added. The specificity of these T clones was then studied by in vitro proliferation assays using as antigens the tetanus toxin, its B fragment (B.fr.) and C fragment (C.fr) obtained from Calbiochem, La Jolla, Ca, USA and the reduced carboxymethylated C fragment (RCM-C. fr).

Typically the proliferation assay was conducted by suspending the T cell clones in wells of a flat bottomed plate in RPMI-c culture medium containing different concentrations of said antigens, in the presence of immortalized or fixed autologous B cells (Lanzavecchia et al. cited heretofore) the purpose of which was to present the antigens.

The B cells were immortalized by known methods with Epstein Barr Virus (EBV) by suspending the PBMC cells in RPMI-c culture medium containing 30% of supernatant of the B95.8 cell line which produces EBV.

The cells were fixed as described by Shimonkevitz R. et al., J. Immunol. 133, 2067 (1984) by suspending them in Hank culture medium (GIBCO, Paisley, Scotland) and fixing them with 0.05% of glutaraldehyde for half a minute. The reaction was blocked by adding RPMI, and the cells were recovered by centrifuging and repeatedly washed.

The proliferation reaction was conducted at 37 ° C for 48 hours in 5% $CO_2$ and the cultures were pulsed with 1 μCi of ($^3$H)-thymidine. On termination of the reaction the number of cells incorporating the radioactivity was determined by a scintillograph. The results, expressed as mean count per minute (cpm) of a double culture, showed that some clones mapped in the TT C-fragment, whereas other clones mapped in the B-fragment.

In accordance with the present invention and in order to more precisely localize those T epitopes in the TT which had been recognised by said clones, in vitro proliferation assays were conducted using as antigens different fragments of RCM-C.fr produced by chemical modification or proteolytic digestion with trypsin and chemotrypsin, with B cells fixed with glutaraldehyde as APC.

Also used as antigens were peptide fragments obtained via recombinant DNA by known methods, by cultivating E. coli cells transformed with a hybrid plasmid containing the nucleotide sequence encoding for said fragments.

Specifically, the following recombinant peptides were prepared: Tet 6 (TT fragment 406-743), Tet 15 (TT fragment 604-1315), Tet 3 (TT fragment 744-1315), Tet 5 (TT fragment 1-405) and Tet 97 (TT fragment 1-535).

The results obtained enabled the sequences recognised by the human T cell clones (T epitopes) in the TT segments 830-843, 830-844 and 1273-1284 of C. fr. to be identified.

In accordance with the present invention, the class II restriction element of the MHC used by the clones was then determined by proliferation assays using as APC an HAR homozygote D3 cell line. The results obtained showed that both clones when in the presence of TT proliferated well, as they did when in the presence of the B KK35 cell line as APC.

Again, in order to determine which of the three molecules DR, DP or DQ of class II of the MHC uas used as the restriction element by the aforesaid clones, proliferation assays were conducted by adding anti-DR, -DP or -DQ monoclonal antibodies (Mabs) to the cultures.

In this manner it was found that only the anti-DR Mabs strongly inhibited proliferation of said clones. This indicated that they used the DE antigen of class II of the MHC as restriction element. In accordance

with the present invention peptides were then synthesized having an amino acid sequence corresponding to that of the T epitopes identified as stated above.

Specifically, the peptides were synthesized, corresponding respectively to the TT segments 1273-1284 and 830-843 and 830-844 having the following amino acid sequence:
Gly-Gln-Ile-Gly-Asn-Asp-Pro-Asn-Arg-Asp-Ile-Leu
(hereunder indicated TT = 1)
Gln-Tyr-Ile-Lys-Ala-Asn-Ser-Lys-Phe-Ile-Gly-ILe-Thr-Glu
(hereunder indicated TT = 2)
Gln-Tyr-Ile-Lys-Ala-Asn-Ser-Lys-Phe-Ile-Gly-ILe-Thr-Glu-Leu and analogs of the TT = 2 characterized by deletions either of the N-terminal portion, or of the C-terminal portion, or by substitutions of aminoacid residues having the following sequences:

Ile-Lys-Ala-Asn-Ser-Lys-Phe-Ile-Gly-ILe-Thr-Glu;

Gln-Tyr-Ile-Lys-Ala-Asn-Ser-Lys-Phe-Ile-Gly-ILe-Thr;

Gln-Tyr-Ile-Lys-Ala-Asn-Ser-Lys-Phe-Ile-Gly-ILe;

Gln-Ala-Ile-Lys-Ala-Asn-Ser-Lys-Phe-Ile-Gly-ILe-Thr-Glu;

Gln-Phe-Ile-Lys-Ala-Asn-Ser-Lys-Phe-Ile-Gly-ILe-Thr-Glu;

Gln-Tyr-Ile-Lys-Ala-Asn-Ser-Glu-Phe-Ile-Gly-ILe-Thr-Glu;

Gln-Tyr-Ile-Glu-Ala-Asn-Ser-Lys-Phe-Ile-Gly-ILe-Thr-Glu;

Gln-Tyr-Ile-Lys-Ala-Ala-Ser-Lys-Phe-Ile-Gly-ILe-Thr-Glu.

Said peptides can be synthesized in solid phase or in homogeneous phase by operating in accordance with one of the known general methods.

Preferably said peptides were synthesized in the solid phase using a commercial polyacrylamide resin, namely 4-hydroxy-methylphenoxy-acetic acid as the peptide-resin reversible connection handle and the fluorenyl-methoxycarbonyl group (Fmoc) as the N-terminal protector group for the amino acid residues in accordance with the strategy reported in Examples 2 and 3.

The reactive functional groups in the side chain of the amino acid residues were protected using protector groups chosen from those generally used in peptide synthesis.

Preferably, the trifluoroacetyl group (TFA) was used for the lysine (Lys), tert-butyl ether ($Bu^t$) for the serine (Ser), threonine (Thr) and tyrosine (Tyr), tert-butyl ester ($OBu^t$) for the glutamic acid (Glu) and the 4-methoxy-2,3,6-trimethylbenzenesulphonyl group (Mtr) for the arginine (Arg).

The suitably protected amino acids were condensed individually with symmetrical anhydrides or esters of pentafluorophenol and/or p-nitrophenol.

The peptide was removed from the resin, in accordance with the present invention, using an aqueous solution of trifluoroacetic acid at ambient temperature (20-25 °C) for the time required to simultaneously remove the protector groups of t-butyl type but not the TFA or Mtr.

This strategy enables peptides to be obtained in which the only free amino group available for its subsequent conjugation with a hapten is the N-terminal group.

The peptides synthesized in this manner were then purified by gel filtration chromatography followed by preparative HPLC in the usual manner.

A measurement was then made of the capacity of the peptides according to the present invention to stimulate in the presence of APC the proliferation of human T cell clones having a different set of class II HLA molecules.

The results showed that said peptides were recognised by the different human T cell clones within the context of numerous alleles of the human major histocompatibility complex.

Consequently said peptides were particularly suitable as universal carriers for the preparation of peptide-hapten immunogenic conjugates.

In this respect they combine within themselves the advantages due to the entire tetanus toxide sequence, ie the capacity to induce the formation of anti-hapten antibodies in most vaccinated individuals, with the advantages relating to their small size (no epitope suppression and more effective control of the conjugation reactions with the hapten).

Examples of haptens which can be bound to said peptide carriers for the preparation of immunogenic conjugates according to the present invention are: peptides or polysaccharides derived from different pathogenic agents such as meningococci, pneumococci, Haemophilus influenzae, B-hemolytic streptococci and Escherichia coli.

Haptens suitable for the purpose of the present invention are also those synthetic peptides having the sequence corresponding to that of one or more B epitopes of a natural antigen.

The conjugation between a peptide carrier according to the present invention and a hapten can be effected using one of the conventional methods employed generally in this particular sector of the art.

Specifically, the conjugation can be conducted using glutaraldehyde as binding agent, as reported for example by Avremas and Ternyck (Immunochemistry 6, 53, 1969).

According to one embodiment of the present invention the peptides TT#1 and TT#2 were used as universal carriers to improve the immunogenicity of synthetic peptides having an amino acid sequence corresponding to that of the immunodominant B epitope of the circumsporoite protein (CSP) of Plasmodium falciparum, which is the etiologic agent of the most serious form of malaria (malignant tertian malaria) in man.

Said synthetic peptides, having the amino acid sequence H-(Asn-Ala-Asn-Pro)$_n$ in which n is between 3 and 40, were in fact not able to induce an anti-CSP antibody response in CBA/ca and DBA/2 mice (non-responder mice) [G. Del Giudice et al., J. Immunol., 137, 2952-2965 (1986)].

In this respect it was shown that (NANP)$_n$ contained within its sequence one or more T epitopes all genetically restricted by the I-A$^b$ allele of the H-2 murine histocompatibility complex present only in C57 BL/6 mice.

Said peptides were prepared as described in Italian patent application 21718 A/85, by polymerizing the activated monomer HCl. H-Asn-Ala-Asn-Pro-OPCP in the presence of triethylamine as basic initiator.

In accordance with the present invention the peptide carrier-(NANP)$_n$ conjugates were prepared by reacting the aldehyde groups of glutaraldehyde with the terminal amino group of the asparagine (Asn) of (NANP)$_n$ and with the terminal amino group of the carrier. The conjugation reaction was implemented in two stages. In the first stage the NANP)$_n$ was left to react in a phosphate buffer (pH 7.4) with an excess of glutaraldehyde ($OHCCH_2CH_2CH_2CHO$) under neutrality conditions to give rise to the formation, by aldol autocondensation, of the polymer with aldehyde functions:

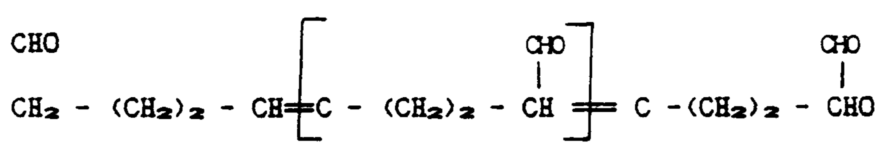

which seems to be the effective conjugation agent.

On termination of the conjugation reaction, the resultant product (NANP)$_n$-glutaraldehyde was purified by gel filtration from the unreacted glutaraldehyde.

In the second stage, the (NANP)$_n$-glutaraldehyde conjugate was reacted in a phosphate buffer (pH 7.4) or in dimethylsulphoxide (DMSO) with a molar excess of the peptide carrier (between 2.5 and 25) with respect to the (NANP)$_n$-glutaraldehyde conjugate, so utilizing the presence of the still free aldehyde groups on the glutaraldehyde conjugated with the (NANP)$_n$ for attacking the terminal amino group of the carrier.

The reaction was conducted at ambient temperature (20-25 °C) under agitation for 3 days, to obtain a yellowish solution.

A reducing substance can be added to said solution, such as $NaBH_4$ which by reducing the bonds (Schiff's bases) between the amino group of the peptides and the aldehyde groups of the glutaraldehyde stabilize the bonds present in the conjugate.

Preferably according to the present invention the $NaBH_4$ was used to give a further margin of safety in the stability of the bond between the two peptides.

The reduction reaction was conducted at ambient temperature for about two hours, on termination of which the formed precipitate was solubilized by adjusting the pH to acid value.

The solution obtained was purified by gel filtration, eluting with 0.1 M acetic acid to separate the formed conjugate from the excess peptide carrier.

One of the conventional methods was then used to release the protector groups for the amine functions in the side chains of the peptide carrier amino acid residues.

According to the present invention, the protector group 4-methoxy-2,3,6-trimethylbenzenesulphonyl of the arginine of the TT#1 peptide was removed by treating the conjugate with a trifluoro-acetic acid/phenol (95/5 v/w) solution, the trifluoroacetyl group of the lysine of the peptide TT#2 being released by treatment with a basic aqueous solution.

The conjugates were then purified by lyophilization and chromatography.

According to the present invention, the immunogenicity of said conjugates was verified by stimulating the in vitro proliferation of human T lymphocytes specific for the peptide carrier in the presence of autologous B cell lines as APC.

In addition a determination was also made of the capacity of said conjugates to induce the production of anti-$(NANP)_n$ antibodies in mice who were non-responders to $(NANP)_n$.

The proliferation results showed both that the long $(NANP)_n$ sequence did not influence the capacity of the peptide carrier to function as a T epitope and that the conjugation reaction had not altered the structure of said T epitope.

In vivo immunization data (mice) also showed the absence of a genetic restriction and an epitope suppression of the carrier. In fact, the mice who were non-responders to $(NANP)_n$ were able to produce a high concentration of anti-$(NANP)_n$ antibodies.

As stated heretofore, said peptide carriers are recognised within the ambit of the very widespread human restriction element DR, and as there is considerable superimposing between the restriction elements of the human histocompatibility complex (HLA) and the murine (H-2), the experiments conducted on the mouse can be considered valid and the results can be extrapolated for man.

In conclusion, the synthetic peptides according to the present invention are suitable as universal carriers for the preparation of peptide carrier-hapten immunogenic conjugates able to induce protective immunity which is not genetically restricted or with only slight genetic restriction against different pathogenic agents.

Particularly preferred as universal carriers are the synthetic peptides corresponding to the regions 830-843 (TT = 2) and 830-844 of the TT, and the analogs of the TT = 2 peptide.

Immunogenic conjugates according to the present invention can be administered to man in a single dose or in successive doses in the form of pharmaceutical composition (vaccines) which contain them in a form and in a quantity suitable for inducing a protective immune response.

The preferred forms are pharmaceutical compositions prepared as suspensions or solutions containing the conjugate, which are easily administered by injection.

If desired, an adjuvant can be added to said compositions to improve their immune response.

The following experimental examples illustrate but do not limit the invention.

EXAMPLE 1

Identification of the T epitopes of tetanus toxin

T cell clones (KT1, KT2, KT4, KT30, KT40 and KT42) were used, obtained from the peripheral blood of a donor (KK) with a DR 3,5 gene set immunized with tetanus toxoid. These clones were stabilized by known methods [Lanzavecchia A., Nature 314 (1985), 357-36] and were kept under culture by periodic restimulation with irradiated mononuclear blood cells of peripheral blood (PBMC) with 1% phytohemagglutination (Gibco, Paisley, Scotland). 5 days after the T blast cells were washed and cultivated in RPMI-c culture medium (consisting of the Gibco RPMI growth medium fed with calf foetal serum (10%) inactivated by heating, 2 mM L-glutamine, 1 mM sodium pyruvate, 5 x $10^{-5}$ M 2-mercaptoethanol and a mixture of non-essential amino acids (1%) of (Gibco) supplemented by 40 units/ml of recombinant human interleukin 2 (Hoffman La Roche).

The specificity of said clones was then studied by the in vitro proliferation assay using as antigens the tetanus toxin, its B fragment (B.fr.) and C fragment (C.fr) of Calbiochem La Jolla, Ca, USA, and the reduced carboxylated C fragment (RCM-C,fr). Practically the reduction of the C fragment (C.fr.) was effected in phosphate buffer saline (PBS) pH 7.0 and 8 M urea and treated with 20 mM dithiothreitol at 37 °C for 30 minutes.

The reduced fragment was then carboxymethylated with 80 mM iodoacetamide for 60 minutes at 4 °C in the dark and then was dialysed extensively against water.

The proliferation assay was then conducted by seeding each well of a flat bottomed 96-well plate (Cluster Cambridge MA) 100 µl of RPMI-c culture medium containing the aforesaid antigens, with 2 x $10^4$ T cell clones in 0.1 ml of RPMI-c culture medium in the presence of said antigens and 2 x $10^4$ EBV-B cells (autologous B lymphocytes transformed with the Epstein-Barr virus treated with mitomycin C) as APC.

The proliferation reaction was conducted at 37 °C for 48 hours in 5% $CO_2$ and the cells were pulsed adding 1 µCi of methyl-($^3$H)-thymidine (185 GBq/mmoles, Amersham International, Amersham, GB).

After about 20 hours the number of cells incorporating radioactivity was determined.

The results expressed as the mean count per minute (cpm) of a double culture, showed that two T cell clones (KT4 and KT30) mapped in the TT C-fragment, whereas the KT2 clone mapped in the B-fragment.

A procedure was then carried out to more precisely localize those T epitopes in the TT which had been recognised by said clones.

Proliferation assays were then conducted operating as stated heretofore, using as antigens different-length fragments of RCM-C.fr produced by enzymatic digestion with trypsin and chemotrypsin (Sigma, St. Louis, MO) or by chemical modification.

In practice the enzymatic reaction was conducted by suspending the fragment in PBS (pH 7.0) containing $Ca^{2+}$ ions and the enzyme for 20 hours at 37 °C using an enzyme-substrate ratio of between 1:1 and 20:1 and separating the fragments obtained.

The chemical treatment was conducted as described by Corradin G. and Harbury H.A., (1970) Biochem.Biophys. Acta, 221, 489.

Peptide fragments obtained via recombinant DNA by known methods, by cultivating E. coli cells engineered by known methods were also used.

Specifically, the following recombinant peptides were prepared and used: Tet 6 (TT fragment 406-743), Tet 15 (TT fragment 604-1315), Tet 3 (TT fragment 744-1315), Tet 5 (TT fragment 1-405) and Tet 97 (TT fragment 1-535).

The results which have been obtained have made it possible to identify, in the C fragment of the TT, the regions 1273-1284, 830-843 and 830-844, corresponding to the T epitopes having the following aminoacid sequences:
Gly-Gln-Ile-Gly-Asn-Asp-Pro-Asn-Arg-Asn-Ile-Leu
(Hereunder indicated TT = 1);
Gln-Tyr-Ile-Lys-Ala-Asn-Ser-Lys-Phe-Ile-Gly-Ile-Thr-Glu
(hereunder indicated TT = 2);
Gln-Tyr-Ile-Lys-Ala-Asn-Ser-Lys-Phe-Ile-Gly-Ile-Thr-Glu-Leu.

EXAMPLE 2

Synthesis of the peptide TT#1

The peptide TT#1 was synthesized in the solid phase with a Beckman 99 B automatic synthesizer using a commercial polyacrylamide resin (PepSyn, Cambridge research Biochemicals) functionalized with norleucine as internal reference amino acid and 4-hydroxymethylphenoxyacetic acid as reversible peptide-resin connection handle.

1 g of said resin with a functionalization of 1.1 milliequivalents/g was swollen in 32 ml of N,N-dimethylformamide (DMF) overnight at ambient temperature (2 -25 °C) and then washed with 1 ml of DMF 1 times (1 minute each time).

The first amino acid residue (Leu), protected at the alpha-amino group by the protector group fluorenylmethoxycarbonyl (Fmoc), was esterified at the resin by the amino acid symmetric anhydride reaction.

In practice, 1.238 g (1.8 mmoles) of $(Fmoc\ Leu)_2O$ were reacted with the resin in 16 ml of DMF in the presence of 0.022 g (0.18 mmoles) of 4-dimethylaminopyridine (DMAP) and 0.198 ml (1.8 mmoles) of N-methylmorpholine (NMM) at ambient temperature for 3 minutes.

On termination of the esterification reaction, the resin was washed 5 times (1 minute each time) with DMF, twice with a piperidine/DMF solution (2/8 v/v) for 3 and 7 minutes to remove the Fmoc protector group and finally 10 times with DMF (1 minute each time).

The other amino acids were then introduced by acylation of the amino acid activated at the carboxyl group and protected at the alpha-amino group with Fmoc on the free $-NH_2$ groups of the growing peptide chain.

The side-chain functions of the amino acids were protected respectively with t-butylester ($OBu^t$) for the aspartic acid and 4-methoxy-2,3,6-trimethylbenzenesulphonyl (Mtr) for the arginine.

The stated washing and Fmoc removal operations were carried out between one acylation reaction and the next.

The acylation reaction was conducted at ambient temperature for 60 minutes.

All the amino acid residues with the exception of the glutamine (Gln) and asparagine (Asn) were introduced using the corresponding symmetrical anhydrides as active form (1.8 mmoles in 16 ml of DMF).

For Gln and Asn, p-nitrophenol ester (1.8 mmoles) and the pentafluorophenol ester (1.8 mmoles) of the amino acid were used in the presence of 0.244 g (1.8 mmoles) of 1-hydroxybenzotriazole (HOBt) respectively.

The symmetrical anhydride of the suitably protected amino acids was prepared immediately before the acylation reaction, by reacting 3.6 mmoles of Fmoc-amino acid with 0.371 g (1.8 mmoles) of dicyclohexylcarbodiimide (DCCI) in 2ml of methylene chloride at ambient temperature for 10 minutes.

On termination of the reaction, the dicyclohexylurea which had formed was separated by filtration, the solvent evaporated and the symmetrical anhydride obtained was recovered.

For each acylation reaction, the completion of the reaction was checked by the ninhydrin test [E. Kaiser et al., Anal. Biochem., 34 (198 ), 595] and the trinitrobenzosulphonic acid test [W.S. Hancock et al., Anal. Biochem., 71, (1976), 261].

The samples, taken after 3 minutes of reaction, gave positive results.

On amino acid analysis, the resin-peptide gave the following results:

| Asp | Glu | Pro | Gly | Ile | Leu | N-leu | Arg |
|---|---|---|---|---|---|---|---|
| 4.00 | 0.98(1) | 0.9(1) | 2.07(2) | 2.06(2) | 1.08(1) | 1.18 | 1.35(1) |

The theoretical values are shown in parentheses.

A part of the peptide TT#1 was preserved on the resin for possible synthesis of peptides linked to TT#1, whereas the remainder was released by treatment at ambient temperature for 2 hours with the trifluoroacetic acid solution ($TFA/H_2O = 90/1$, v/w).

Said solution removes the t-butylester protection group but not the 4-methoxy-2,3,6-trimethylbenzensulphonyl group.

This strategy enabled the peptide to be obtained in which the only amino group useful for its conjugation with a hapten is the terminal glycine group.

These chromatographic conditions were considered suitable for preparative HPLC.

In practice a column of dimensions 24 x 0.2 cm was used filled with RP-18 silica (25-4 $\mu$m) (ICN BIOMEDICALS, West Germany), using an aqueous 0.1% TFA solution containing 29% of acetonitrile as eluent.

Various chromatograph runs were carried out varying both the quantity of feed peptide and the percentage of acrylonitrile in the eluent in an attempt to optimize the pure produce yield.

The following table I shows the yields obtained as a function of the various parameters.

TABLE I

| Preparation No. | % Acetonitrile | Feed (mg) | Purified product mg | Yield % |
|---|---|---|---|---|
| 1 | 29 | 25 | 7.27 | 29 |
| 2 | 27 | 25 | 9.67 | 39 |
| 3 | 27 | 12.5 | 4.28 | 34 |
| 4 | 27 | 25 | 9.34 | 37 |
| 5 | 27 | 50 | 18.36 | 37 |

EXAMPLE 3

Synthesis of the peptide TT#2

The peptide sythesis was conducted in accordance with the Fmoc polyamide strategy described in Example 2 using the flow variant of the same method [E. Atherton et al., Tetrahedron 44 (1968), 843-887; A. Bryland and R.C. Sheppard, ibid. 859-876].

Esterification of the first amino acid residue (Glu) at the polymer support was conducted using the asymmetric anhydride of the protected amino acid [Fmoc-Glu $(OBu^t)]_2O$ in the presence of the catalyst 4-N, N'-dimethylaminopyridine (0.1 mmoles, 0.012 g) and N-methylmorpholine (1 mmole, 110 µl).

Said anhydride was obtained by reacting Fmoc-Glu $(OBu^t)OH$ (2 mmoles, 0.851 g) with DCCI (1 mmole, 0.206 g).

The reaction was conducted on 3 g of the commercial resin ULTROSYN (Pharmacia-LKB, Sweden) dissolved in 3 ml of DMF, at ambient temperature for 0.5 hours.

The functionalization was 80%.

The resin was then washed with DMF and packed into two omnifit columns (10 x 1.0 cm) (1.5 g of resin per column) forming part of the PHARMACIA-LKB flow synthesizer model 4170.

The synthesis strategy was that described in the cited works.

The protector groups used for the amino acid side-chain functions were trifluoroacetyl (TFA) for the lysine (Lys), tert-butyl ether $(Bu^t)$ for Ser, Thr and Tyr, and tert-butyl ester $(OBu^t)$ for Glu, The reversible connection handle was 4-hydroxymethylphenoxyacetate.

The peptide was released from the resin by treatment with trifluoroacetic acid/$H_2O$ (95/5, v/v) for 1.5 hours at ambient temperature. Yield 93%.

This treatment removes all the tert-butyl type protections but leaves the TFA protector group on the lysine residues intact. The resultant peptide thus possesses a single amino function available at the terminal amino residue.

The peptide was then purified by gel filtration using dimethylsulphoxide (DMSO) as eluent, and an 80 x 2.6 cm column packed with Sephadex G-10 resin and eluted at a throughput of 36 ml/hour.

The fractions corresponding to the material eluted at the column head were collected, diluted with water and lyophilized.

The amino acid analysis of the purified peptide gave the following result (theoretical values in parentheses).

| Asp | Thr | Ser | Glu | Gly | Ala |
|---|---|---|---|---|---|
| 1.06(1) | 1.03(1) | 0.98(1) | 1.80(2) | 0.95(1) | 1.08(1) |

| Ile | Tyr | Phe | Lys |
|---|---|---|---|
| 2.81(3) | 1.00(1) | 1.00(1) | 2.32(2) |

EXAMPLE 4

Synthesis of the 830-844 peptide.

The synthesis of the peptide has been carried out by operating as in the previous Example 3, in which the esterification of the first aminoacid residue (Leu) at the Polymeric substrate has been effected by employing the symmetrical anhydride of the protected aminoacid $(Fmoc\text{-}Leu)_2O$.

The aminoacid analysis of the purified peptide has given the following result (theoretical values in parentheses):

| Asp | Thr | Ser | Glu | Gly | Ala | Ile |
|---|---|---|---|---|---|---|
| 1.06(1) | 1.03(1) | 0.98(1) | 1.80(2) | 0.95(1) | 1.08(1) | 2.81(3) |

| Tyr | Phe | Lys | Leu |
|---|---|---|---|
| 1.00(1) | 1.00(1) | 2.32(2) | 0.95(1). |

EXAMPLE 5

Isolation of T-cellular clones which are specific for the TT=2 peptide.

Mononucleated cells of peripheral blood (PBMC) isolated from different donors (Typized HLA) which had been immunized with the tetanus toxoid, have been cultured at a concentration of 7 x $10^5$ in 200 $\mu$l of RPMI-HS culturing medium (RPMI=RPMI 1640 supplemented by 2mM of glutamine, 1% of non-essential aminoacids, 1% of sodium pyruvate, 50 $\mu$g/ml of kanamicin (Flow, Irvine, Scotland); HS=human serum (Swiss red Cross, berne)) in the presence (10 $\mu$M) or in the absence of the TT=2 peptide in microplates having 96 planar-bottomed wells, 6 days after there have been added in each well 30 Units/ml of Interleuchin-2 (IL-2) (Hoffmann La Roche, Nutley, N.J.), and, after 4 additional days, the cultures have ben examined to detect the possible cellular proliferation. The positive cultures have then been expanded in the same culturing medium which had been supplemented by IL-2 and tested for their ability to recognize the TT=2 peptide. A portion of said T cells have been transferred into wells of microplates having 96 planar-bottomed wells, thrice washed and slurried again in 200 $\mu$l of RPMI culturing medium supplemented by the 10% of calf foetal serum (FCS) (Gibco, Paisley, Scotland) in the presence (20 $\mu$M) or in the absence of the TT=2 peptide. Inasmuch as the activated human T-cells express molecules of the II class, they are capable of presenting the peptide the one to the other, the result being a visible agglutination after 6 hours at 37°C. The positive cultures have been cloned by limiting dilution and the clones which are specific for the TT=2 peptide have been isolated and maintained in culture by periodic restimulation with irradiated allogenic PBMC and phytohaemoagglutinin (1%) (Gibco), as described by Lanzavecchia et al., (1988), Nature 334, 530.

A single specific clone only was stored for each positive culture.

As reported in Table II, the clones which are specific for TT=2 were easily isolated from all the donors, irrespective of their DR type. The approximate occurrence of the cells which were specific for the TT=2 was comprised between 1 in 3 x $10^4$ and 1 in 3 x $10^5$, and represented but the 5%, or less, of all the T cells which were specific for the Tetanus Toxoid (TT).

TABLE II

| T-cell response to TT = 2 | | |
|---|---|---|
| Donors (DR) (a) | Isolated clones | Restriction (b) |
| KK(3,5) | 1 | DR5 |
| GC(5) | 21 | DR5 |
| GAR(1,5) | 3 | DR5 |
| PP(2,6) | 3 | DR? |
| FS(1,8) | 7 | DR? |
| DP(1,4) | 2 | DR4 |
| SP(7) | 3 | DR7 |
| GU(3,7) | 6 | DR7 |
| BDM(5) | 9 | DR5 |
| KS(4) | 6 | DRw4(4) |
| ZU(3,4) | 9 | DR3/DRw52b |
| AL(6) | 3 | DR6/DR |
| BR(4) | 4 | DRw4(4)/DR |
| MG(2,9) | 7 | DR9/DR |
| GM(1,2) | 6 | DR1/DR |

where:

(a) = PBMC from different DR-typized donors;

(b) = indicates all the II-class alleles of the class MHC which might present the peptide to at least one T-cellular clone.

All of the isolated clones proliferate responsively both to the TT = 2 peptide, and the entire molecule of the Tetanus Toxoid presented by autologous APC cells, thus evidencing their specificity towards TT.

These results show that TT = 2 is universally recognized after immunization with TT and that it is capable of becoming associated to different II-class molecules.

Example 6

Characterization of the T-cellular clones which are specific for TT = 2.

A) Determination of the restriction pattern of the T-cells.

In order to determine the isotype of the II-class molecules which have been recognized by every T-cellular clone, there have been conducted proliferation tests by using anti-DR monoclonal antibodies as obtained from the ATCC L243 hibridome, which is available by the Americal Type Culture Center, anti-DP monoclonal antibodies, and anti-DQ monoclonal antibodies.

In actual practice, the T-cells have been cultured with autologous EBV-B cells in the presence of limiting concentrations of both TT = 2 and monoclonal anti-DR antibodies (L243, 1:4 of the culture supernatant), anti-DP and anti-DQ antibodies, both as 1:1000 ascytes.

The results show that the clones which specific for TT = 2 are restricted DRs, since only the anti-DR antibodies inhibit the cellular proliferation. In order to identify the DR restriction alleles, each clone has been tested for its capacity of proliferating in response to a group of allogenic EPBV-B HLA homozygote cells (used as APC), which have been pulsated, or not, with the TT = 2 peptide for 2 hours at 37 °C, washed 4 times and irradiated.

The results show that a large number of DR alleles can be used as restriction molecules. These latter include DR1-9, 4 x 4, 5JVM and DRw52b (Table III).

TABLE III

Proliferation response of T-cellular clones (cpm x $10^{-3}$) (a)

| p2-labeled APC (DR) (b) | GAR9.2 (1,5) (c) | ZU10.5 (3,4) | KS9.6 (4w4) | SP2.2 (7) | ZU5.4 (3,4) | MG18.8 (9,2) | AL4.1 (6) | GH2.11 (1,2) |
|---|---|---|---|---|---|---|---|---|
| EDR (1) | 0 | 0 | 0 | 0 | 0 | 0 | 0 | 116(.1) |
| NOL (w15(2)) | 0 | 0 | 0 | 0 | 0 | 0 | 0 | 120(.2) |
| AVL (w18(3),w52a) | 0 | 41(.02) | 0 | 0 | 0 | 0 | 0 | 60(2) |
| BSM (w4(4)) | 0 | 0 | 45(1) | 0 | 0 | 0 | 0 | 43(.1) |
| ATH (w11(5),w52b) | 111(.02)(d) | 0 | 0 | 0 | 0 | 117(.1) | 0 | 2 |
| JVM (w11JVM,w52b) | 0 | 0 | 0 | 0 | 112(.1) | 0 | 154(.1) | 2 |
| HHK (w13(w6),W52a) | 0 | 0 | 0 | 0 | 0 | 0 | 97(.5) | 132(2) |
| APD (w13(w6),w52b) | 0 | 0 | 0 | 0 | 91(.1) | 0 | 123(.1) | 1 |
| KRA (w13(w6),W52c) | 0 | 0 | 0 | 0 | 0 | 0 | 113 | 0 |
| EKR (7,w53) | 0 | 0 | 0 | 21(1) | 0 | 0 | 0 | 40(.7) |
| LUY (8.3) | 0 | 0 | 0 | 0 | 0 | 39(10) | 0 | 115(10) |
| DKB (9.w53) | 0 | 0 | 0 | 0 | 0 | 95(.01) | 0 | 50(5) |

(a) Different clones were tested for their capacity of proliferating in the presence of APC presenting the TT=2 antigene. The response was inhibited by anti-DR antibodies only.

(b) indicates the DR-typization of the APC.

(c) indicates the DR-typization of the donor from whom the clones had been isolated.

(d) indicates the concentration, in micromols, of the peptide which is capable of inducing the 30% of the maximum response.

The dose-response plot as determined for each clone, shows that all the clones react in the field of very low concentrations of the peptide (0.1-2 μM) (Table III). This fact is an evidence that all the clones possess the same high affinity, and thus all the DR alleles are capable of presenting the peptide with the same efficiency.

Quite surprisingly, it has been ascertained that a few clones (defined as monogamic) recognize the TT = 2 in association with a single DR allele, whereas other clones recognize in association with two or more alleles. Examples are the clone GM2.11, which recognizes TT = 2 in association with DR1, 2, 4w4 and 7, and the clone AL4.1, which recognizes TT = 2 in association with DR5JVM and 6.

B) Identification of the minimum antigenic sequence recognized by the T-cells

In order to identify a minimum antigenic sequence in the interior of the TT = 2 peptide, proliferation tests have been carried out by exploiting a series of TT = 2 deletion fragments devoid of the N-terminal, or the C-terminal region.

Moreover, TT = 2 analogs have been synthesized, which are characterized in that an aminoacid residue had been substituted. The proliferation results which have been obtained by exploiting as APC different T-cellular clones show that TT = 2 contains a single 831-842 epitope, which is recognized in the context of each of the assayed molecules (from DR1 to 9, 52a and 52b) and that the TT = 2 deletion peptides and those which contain the substituted aminoacid residue have a similar effect on the recognization by different T-cellular clones (Table IV).

TABLE IV

Peptide concentration (μM) required to induce the 30% of the maximum response (a)

| | T cell clone: | AL4.1 | GAR9.2 | ZU10.5 | MG18.8 | ZU5.4 | GM2.11 | GM2.11 | GM2.11 | GM2.11 |
|---|---|---|---|---|---|---|---|---|---|---|
| 830 844 | APC (DR) [b]: | APD(6) | ATH(5) | AVL(3) | DKB(9) | ATH(w52b) | OOS(1) | NOL(2) | BSM(4) | PITOUT(7) |
| QYIKANSKFIGITEL | | <0.1 | 0.02 | 0.02 | 0.02 | 0.02 | 0.02 | 0.2 | 0.1 | 0.01 |
| Y------------- | | 1 | 10 | 5 | 1 | 10 | 0.5 | 0.2 | 10 | 0.1 |
| I------------ | | >10 | >10 | >10 | >10 | >10 | >10 | >10 | >10 | >10 |
| -------------T | | 2.5 | 10 | 5 | 5 | 10 | 2 | 1 | 10 | 0.2 |
| ------------I | | >10 | >10 | >10 | >10 | >10 | >10 | >10 | >10 | >10 |
| -A------------- | | >10 | >10 | >10 | >10 | >10 | >10 | >10 | >10 | >10 |
| -F------------- | | >10 | >10 | >10 | >10 | >10 | >10 | >10 | >10 | >10 |
| --------E------ | | >10 | >10 | >10 | >10 | >10 | >10 | >10 | >10 | >10 |
| ---E----------- | | >10 | >10 | >10 | >10 | >10 | >10 | >10 | >10 | >10 |
| -----A--------- | | >10 | 1 | >10 | >10 | >10 | >10 | >10 | >10 | >10 |

(a) monogamic and promiscuous clones were assayed for their capability of proliferating with APC which had been pulsated with different concentrations of peptides or substitutes.

(b) the restriction list of the assayed clone is reported inparentheses.

EXAMPLE 7

Synthesis of $(NANP)_n$-TT#1 conjugates

The peptide TT#1 protected on the arginine guanidino group with the Mtr group and obtained as described in Example 2 was used in the synthesis of the following conjugates:

1) $(Asn\text{-}Ala\text{-}Asn\text{-}Pro)_{40}$-TT#1 or $(NANP)_{40}$-TT#1

2) $(Asn\text{-}Ala\text{-}Asn\text{-}Pro)_{20}$-TT#1 or $(NANP)_{20}$-TT#1

The polymers $(NANP)_{40}$ and $(NANP)_{20}$ were obtained by polymerizing the activated monomer Hcl.H-AsnAlaAsnPro-OCPP in the presence of triethylamine as basic initiator as described in Italian patent application 21718 A/85.

a) Synthesis of $(NANP)_{40}$-TT#1

40 mg of $(NANP)_{40}$ dissolved in 2.5 ml of phosphate buffer solution of pH 7.44 were transferred to a reactor of 3 ml in volume, containing 52 µl (260 meq) of an aqueous 5% glutaraldehyde solution (FluKa), corresponding to a 100 times excess over the $(NANP)_{40}$.

The reaction mixture was kept under gentle stirring at ambient temperature overnight.

The solution was then chromatographed in an 85 x 2.5 cm column with Sephadex G-25 filling, eluting with 0.1 M acetic acid.

The fractions corresponding to the peak which elutes with the empty volume of the column corresponding to $(NANP)_{40}$ bound to the glutaraldehyde were then collected and lyophilized.

2.5 ml of phosphate buffer (pH 7.4) containing the $(NANP)_{40}$-glutaraldehyde were added to a 3 ml reactor containing 9.67 mg (6.32 mmoles) of TT#1. The molar excess of TT#1 over the $(NANP)_{40}$-glutaraldehyde was about 2.5 times.

The yellow solution obtained was left stirring for 3 days. On termination of the reaction 25 µg of $NaBH_4$ reducer were added. The resultant solution was stirred for a further two hours to obtain a suspended precipitate.

This precipitate was dissolved by adjusting the pH to about 4.0 by adding 1 M acetic acid.

The solution was then purified by gel filtration on Sephadex G-25 eluting with 0.1 M acetic acid to separate the formed conjugate from the excess TT#1.

The conjugation reaction yield, calculated by amino acid analysis, was 17%.

The 4-methoxy-2,3,6-trimethylbenzenesulphonyl protector group for the arginine of the TT#1 peptide was then removed by treating the conjugate with 5 ml of TFA/phenol (95/5, v/w) for 5 hours.

The solution was evaporated to dryness under vacuum and the residue obtained was dissolved in 5ml of $H_2O$, transferred to a 25 ml separator funnel and extracted twice with ethyl ether.

The ether phase was re-extracted with water and the recovered aqueous phases were pooled and lyophilized.

b) Synthesis of $(NANP)_{20}$-TT#1

The procedure for preparing this conjugate was analogous to that of point a) but using in the second stage of the reaction a 25 times excess of TT#1 over the $(NANP)_{20}$-glutaraldehyde to increase the conjugate reaction yield.

Amino acid analysis showed this yield to be 32%.

EXAMPLE 8

Synthesis of the conjugate $(NANP)_{20}$-TT#2

The procedure was the same as that used for the conjugate $(NANP)_{20}$-TT#1, using as conjugate reaction solvent 5 ml of dimethylsulphoxide (DMSO) instead of the phosphate buffer, together with 100 mg of TT#2 and 52 mg of $(NANP)_{20}$-glutaraldehyde. 50 mg of $NaBH_4$ were also used.

On termination of the conjugation reaction, the resultant mixture was chromatographed in a column (80 x 2.6 cm) of Sephadex G-25, eluting with 0.1 M $CH_3COOH$.

The fractions corresponding to the material eluted at the head of the solvent were collected and lyophilized.

The lyophilate was then treated with 25 ml of 1 m aqueous piperidine solution at ambient temperature for 2 hours.

The solution obtained was then neutralized to pH 4 with dilute acetic acid and then again fed to the Sephadex G-25 column and eluted with 0.1 M acetic acid.

The fractions corresponding to the material eluted at the head were collected and lyophilized.

Amino acid analysis of the $(NANP)_{20}$-TT#2 conjugate indicates a conjugation yield of 75%.

EXAMPLE 9

Use of the conjugate $(NANP)_{20}$-TT#2 for the in vivo production of anti-NANP antibodies

The experiment was carried out using 3 strains of mice:

- C57/8L/6 mice, who are natural responders to $NANP_{40}$
- CB4/ca and DBA/2 mice, who are non-responders to $NANP_{40}$

The object of the experiment is to show that if the TT=2 peptide is covalently bonded to $(NANP)_{20}$ is able to increase the number of strains of mice able to respond to the antigen NANP.

50 µg of $(NANP)_{20}$-TT#2 conjugate dissolved in a final volume of 50 µl of complete Freund's adjuvant (CFA) were injected at the base of the tail of said mice.

After 3 weeks, in the above manner 25 µg of conjugate emulsified in 25 µl of incomplete Freund's adjuvant (ICFA) were injected (booster dose). Blood was withdrawn from each mouse by pricking the retro-orbital plexus 10 days after the booster injection.

The sera were then tested in an immunoenzymatic ELISA assay using $(NANP)_{40}$ as antigen as described by G. Del Giudice et al., J. Clin., Microbiol., 25 (1987), 91-96.

Figure 1, in which the vertical axis represents optical density and the horizontal axis represents the serum dilutions, shows that both the C57 8L/6 mice and the CPA/ca mice produced high anti-NANP antibody counts, whereas the DRA/2 mice were still non-responders to the conjugate.

EXAMPLE 10

In vitro proliferation of human T lymphocytes using the conjugates $(NANP)_{20}$-TT#1 and $(NANP)_{40}$-TT#1

A flat bottomed 96-well polystyrene plate (Cluster, Cambridge) was used for the proliferation assay.

Each well was seeded with 2 x $10^4$ human T lymphocytes specific for TT#1 in 0.1 ml of RPMI-c culture medium consisting of RPMI growth medium (Gibco, Paisley, Scotland)fed with calf foetal serum (10%) inactivated by heating, 2 mM L-glutamine, 1 mM sodium pyruvate, 5 x $10^{-5}$ M 2-mercaptoethanol and a mixture of non-essential amino acids (1%) of Gibco.

The T cells were incubated at 37°C for 3 days in a 5% $CO_2$ atmosphere in the presence of different concentrations of the two conjugates (Figure 2) and 2 x $10^4$ EBV-B cells (autologous B lymphocytes transformed with the Epstein Barr virus treated with mitomycin C), prepared as described by Lanzavecchia (1985) as previously cited.

The purpose of the EBV-B cells is to present the antigens and stimulate the proliferation of the T clones specific for them.

On the second day all the cultures were pulsed with 1 µCi of ($^3$H) thymidine for 16-18 hours.

On termination of said period the cells were collected on glass fibre filters by means of a cell collector (Skatron, Lier, Norway).

The radioactivity incorporated in said cells was then determined by a scintillograph in terms of the count of cells showing radioactivity.

The results of the proliferation assay are presented as the mean of the counts per minute (cpm) of a double culture plus standard deviation (Figure 2).

The incorporated radioactivity expressed as the ratio:

$$\frac{\text{cpm of cultures with antigen}}{\text{cpm of same cultures without antigen}}$$

increases specifically as the antigen concentration increases.

Operating in this manner it was found that all the tested T lymphocytes proliferated in the presence of the $(NANP)_{40}$-TT#1 and $(NANP)_{20}$-TT#1 conjugates, showing that conjugation with the B epitope of the CSP protein $(NANP)_n$ had not abolished the capacity of the TT#1 peptide to function as the T epitope.

## Claims

### Claims for the following Contracting States : AT, BE, CH, DE, FR, GB, LI, LU, NL, SE

**1.** Synthetic peptides capable of inducing the proliferation of human Th lymphocytes in association with different molecules of the human Major histocompatibility Complex, having the following aminoacid sequence:

H-Gly-Glu-Ile-Gly-Asn-Asp-Pro-Asn-Arg-Asp-Ile-Leu-OH;

H-Gln-Tyr-Ile-Lys-Ala-Asn-Ser-Lys-Phe-Ile-Gly-Ile-Thr-Glu-OH;

H-Gln-Tyr-Ile-Lys-Ala-Asn-Ser-Lys-Phe-Ile-Gly-ILe-Thr-Glu-Leu-OH;

H-Lys-Ala-Asn-Ser-Lys-Phe-Ile-Gly-ILe-Thr-Glu-OH;

H-Gln-Tyr-Ile-Lys-Ala-Asn-Ser-Lys-Phe-Ile-Gly-ILe-Thr-OH;

H-Gln-Tyr-Ile-Lys-Ala-Asn-Ser-Lys-Phe-Ile-Gly-ILe-OH;

H-Gln-Ala-Ile-Lys-Ala-Asn-Ser-Lys-Phe-Ile-Gly-ILe-Thr-Glu-OH;

H-Gln-Phe-Ile-Lys-Ala-Asn-Ser-Lys-Phe-Ile-Gly-ILe-Thr-Glu-OH;

H-Gln-Tyr-Ile-Lys-Ala-Asn-Ser-Glu-Phe-Ile-Gly-ILe-Thr-Glu-OH;

H-Gln-Tyr-Ile-Glu-Ala-Asn-Ser-Lys-Phe-Ile-Gly-ILe-Thr-Glu-OH;

H-Gln-Tyr-Ile-Lys-Ala-Ala-Ser-Lys-Phe-Ile-Gly-ILe-Thr-Glu-OH.

**2.** An immunogenic conjugate consisting of a synthetic peptide carrier covalently bound to a natural or synthetic peptide or polysaccharide hapten derived from a pathogenic agent of interest, wherein said peptide carrier has the amino acid sequence:

H-Gly-Glu-Ile-Gly-Asn-Asp-Pro-Asn-Arg-Asp-Ile-Leu-OH;

H-Gln-Tyr-Ile-Lys-Ala-Asn-Ser-Lys-Phe-Ile-Gly-Ile-Thr-Glu-OH;

H-Gln-Tyr-Ile-Lys-Ala-Asn-Ser-Lys-Phe-Ile-Gly-ILe-Thr-Glu-Leu-OH;

H-Lys-Ala-Asn-Ser-Lys-Phe-Ile-Gly-ILe-Thr-Glu-OH;

H-Gln-Tyr-Ile-Lys-Ala-Asn-Ser-Lys-Phe-Ile-Gly-ILe-Thr-OH;

H-Gln-Tyr-Ile-Lys-Ala-Asn-Ser-Lys-Phe-Ile-Gly-ILe-OH;

H-Gln-Ala-Ile-Lys-Ala-Asn-Ser-Lys-Phe-Ile-Gly-ILe-Thr-Glu-OH;

H-Gln-Phe-Ile-Lys-Ala-Asn-Ser-Lys-Phe-Ile-Gly-ILe-Thr-Glu-OH;

H-Gln-Tyr-Ile-Lys-Ala-Asn-Ser-Glu-Phe-Ile-Gly-ILe-Thr-Glu-OH;

H-Gln-Tyr-Ile-Glu-Ala-Asn-Ser-Lys-Phe-Ile-Gly-ILe-Thr-Glu-OH;

H-Gln-Tyr-Ile-Lys-Ala-Ala-Ser-Lys-Phe-Ile-Gly-ILe-Thr-Glu-OH.

3. An immunogenic conjugate as claimed in claim 2, wherein the hapten is derived from viruses or bacteria.

4. An immunogenic conjugate as claimed in claim 3, wherein the hapten is a polysaccharide molecule derived from pneumococci, meningococci, Haemophilus influenzae, Escherichia coli, and - hemolytic streptococci.

5. An immunogenic conjugate as claimed in claim 2, wherein the hapten is a synthetic peptide of sequence:

H-(Asn-Ala-Asn-Pro)$_n$-OH

where n is between 3 and 40.

6. Immunogenic conjugate claimed in claim 2, useful for preparing synthetic vaccines able to induce a protective antibody response towards a pathogenic agent of interest which is not genetically restricted or is only slightly genetically restricted.

7. Immunogenic conjugate claimed in claim 5, useful for preparing an antimalaria vaccine able to induce a protective antibody response towards infections caused by Plasmodium falciparum which is not genetically restricted or is only slightly genetically restricted.

8. A synthetic vaccine able to induce a protective antibody response in man towards a pathogenic agent which is not genetically restricted or is only slightly genetically restricted, comprising (a) an immunologically effective quantity of the conjugate claimed in claim 2, and (b) a physiologically acceptable medium, and (c) possibly an adjuvant.

9. A synthetic antimalaria vaccine able to induce a protective antibody response in man towards Plasmodium falciparum, which is not genetically restricted or is only slightly genetically restricted, comprising an immunologically effective quantity of the conjugate claimed in claim 5.

**Claims for the following Contracting States : ES, GR**

1. Process for preparing synthetic peptides capable of inducing the proliferation of human Th lymphocytes in association with different molecules of the human Major histocompatibility Complex, having the following amino acid sequence:

H-Gly-Glu-Ile-Gly-Asn-Asp-Pro-Asn-Arg-Asp-Ile-Leu-OH;

H-Gln-Tyr-Ile-Lys-Ala-Asn-Ser-Lys-Phe-Ile-Gly-Ile-Thr-Glu-OH;

H-Gln-Tyr-Ile-Lys-Ala-Asn-Ser-Lys-Phe-Ile-Gly-ILe-Thr-Glu-Leu-OH;

H-Lys-Ala-Asn-Ser-Lys-Phe-Ile-Gly-ILe-Thr-Glu-OH;

H-Gln-Tyr-Ile-Lys-Ala-Asn-Ser-Lys-Phe-Ile-Gly-ILe-Thr-OH;

H-Gln-Tyr-Ile-Lys-Ala-Asn-Ser-Lys-Phe-Ile-Gly-ILe-OH;

H-Gln-Ala-Ile-Lys-Ala-Asn-Ser-Lys-Phe-Ile-Gly-ILe-Thr-Glu-OH;

H-Gln-Phe-Ile-Lys-Ala-Asn-Ser-Lys-Phe-Ile-Gly-ILe-Thr-Glu-OH;

H-Gln-Tyr-Ile-Lys-Ala-Asn-Ser-Glu-Phe-Ile-Gly-ILe-Thr-Glu-OH;

H-Gln-Tyr-Ile-Glu-Ala-Asn-Ser-Lys-Phe-Ile-Gly-ILe-Thr-Glu-OH;

H-Gln-Tyr-Ile-Lys-Ala-Ala-Ser-Lys-Phe-Ile-Gly-ILe-Thr-Glu-OH

characterized in that it comprises the steps of synthesizing the expected synthetic peptide in solid phase, in an automatic synthesizer, in the presence of a commercial polyacrylamide resin, by introducing through an acylation reaction, the amino acids forming the expected sequence one at a time, using as active form the corresponding symmetrical anhydrides or a corresponding ester, after having protected, wherever present, the N-terminal group and the reactive functional groups in the side chain, removing the as formed peptide from the resin and purifying it.

**2.** Process for preparing an immunogenic conjugate characterized in that said conjugate is prepared by the conjugation between a synthetic peptide carrier having one of the following amino acid sequences:

H-Gly-Glu-Ile-Gly-Asn-Asp-Pro-Asn-Arg-Asp-Ile-Leu-OH;

H-Gln-Tyr-Ile-Lys-Ala-Asn-Ser-Lys-Phe-Ile-Gly-Ile-Thr-Glu-OH;

H-Gln-Tyr-Ile-Lys-Ala-Asn-Ser-Lys-Phe-Ile-Gly-ILe-Thr-Glu-Leu-OH;

H-Lys-Ala-Asn-Ser-Lys-Phe-Ile-Gly-ILe-Thr-Glu-OH;

H-Gln-Tyr-Ile-Lys-Ala-Asn-Ser-Lys-Phe-Ile-Gly-ILe-Thr-OH;

H-Gln-Tyr-Ile-Lys-Ala-Asn-Ser-Lys-Phe-Ile-Gly-ILe-OH;

H-Gln-Ala-Ile-Lys-Ala-Asn-Ser-Lys-Phe-Ile-Gly-ILe-Thr-Glu-OH;

H-Gln-Phe-Ile-Lys-Ala-Asn-Ser-Lys-Phe-Ile-Gly-ILe-Thr-Glu-OH;

H-Gln-Tyr-Ile-Lys-Ala-Asn-Ser-Glu-Phe-Ile-Gly-ILe-Thr-Glu-OH;

H-Gln-Tyr-Ile-Glu-Ala-Asn-Ser-Lys-Phe-Ile-Gly-ILe-Thr-Glu-OH;

H-Gln-Tyr-Ile-Lys-Ala-Ala-Ser-Lys-Phe-Ile-Gly-ILe-Thr-Glu-OH

and a natural or synthetic peptide or polysaccharide hapten derived from a pathogenic agent of interest.

**3.** Process according to claim 2, wherein the hapten is derived from viruses or bacteria.

**4.** Process according to claim 3 wherein the hapten is a polysaccharide molecule derived from Pneumococci, Meningococci, Haemophilus influenzae, Escherichia coli and Haemolytic streptococci.

**5.** Process according to claim 2, wherein the hapten is a synthetic peptide of sequence:

H-(Asn-Ala-Asn-Pro)$_n$-OH

where n is between 3 and 40.

**6.** Process for preparing a synthetic vaccine able to induce a protective antibody response in man towards a pathogenic agent which is not genetically restricted or is only slightly genetically restricted, characterized by combining together a) an immunologically effective quantity of the conjugate prepared according to claim 2 and b) a physiologically acceptable medium and c) possibly an adjuvant.

**7.** Process for preparing a synthetic antimalarial vaccine able to induce a protective antibody response in man towards Plasmodium falciparum which is not genetically restricted or is only slightly genetically restricted, comprising an immunologically effective quantity of the conjugate prepared according to claim 5.

**Patentansprüche**
**Patentansprüche für folgende Vertragstaaten : AT, BE, CH, DE, FR, GB, LI, LU, NL, SE**

1. Synthetische, zum Induzieren der Proliferation von Human-Th-Lymphozyten in Assoziation mit verschiedenen Molekülen des humanen Histokompatibilitäts-Hauptkomplexes befähigte Peptide, mit der nachstehenden Aminosäuresequenz:

H-Gly-Glu-Ile-Gly-Asn-Asp-Pro-Asn-Arg-Asp-Ile-Leu-OH;
H-Gln-Tyr-Ile-Lys-Ala-Asn-Ser-Lys-Phe-Ile-Gly-Ile-Thr-Glu-OH;
H-Gln-Tyr-Ile-Lys-Ala-Asn-Ser-Lys-Phe-Ile-Gly-ILe-Thr-Glu-Leu-OH;
H-Lys-Ala-Asn-Ser-Lys-Phe-Ile-Gly-ILe-Thr-Glu-OH;
H-Gln-Tyr-Ile-Lys-Ala-Asn-Ser-Lys-Phe-Ile-Gly-ILe-Thr-OH;
H-Gln-Tyr-Ile-Lys-Ala-Asn-Ser-Lys-Phe-Ile-Gly-ILe-OH;
H-Gln-Ala-Ile-Lys-Ala-Asn-Ser-Lys-Phe-Ile-Gly-ILe-Thr-Glu-OH;
H-Gln-Phe-Ile-Lys-Ala-Asn-Ser-Lys-Phe-Ile-Gly-ILe-Thr-Glu-OH;
H-Gln-Tyr-Ile-Lys-Ala-Asn-Ser-Glu-Phe-Ile-Gly-ILe-Thr-Glu-OH;
H-Gln-Tyr-Ile-Glu-Ala-Asn-Ser-Lys-Phe-Ile-Gly-ILe-Thr-Glu-OH;
H-Gln-Tyr-Ile-Lys-Ala-Ala-Ser-Lys-Phe-Ile-Gly-ILe-Thr-Glu-OH.

2. Immunogenes Konjugat, bestehend aus einem synthetischen Peptidträger, der covalent an ein natürliches oder synthetisches Peptid- oder Polysaccharidhapten gebunden ist, das von einem interessierenden pathogenen Mittel abgeleitet ist, worin dieser Peptidträger die nachstehende Aminosäuresequenz aufweist:

H-Gly-Glu-Ile-Gly-Asn-Asp-Pro-Asn-Arg-Asp-Ile-Leu-OH;
H-Gln-Tyr-Ile-Lys-Ala-Asn-Ser-Lys-Phe-Ile-Gly-Ile-Thr-Glu-OH;
H-Gln-Tyr-Ile-Lys-Ala-Asn-Ser-Lys-Phe-Ile-Gly-ILe-Thr-Glu-Leu-OH;
H-Lys-Ala-Asn-Ser-Lys-Phe-Ile-Gly-ILe-Thr-Glu-OH;
H-Gln-Tyr-Ile-Lys-Ala-Asn-Ser-Lys-Phe-Ile-Gly-ILe-Thr-OH;
H-Gln-Tyr-Ile-Lys-Ala-Asn-Ser-Lys-Phe-Ile-Gly-ILe-OH;
H-Gln-Ala-Ile-Lys-Ala-Asn-Ser-Lys-Phe-Ile-Gly-ILe-Thr-Glu-OH;
H-Gln-Phe-Ile-Lys-Ala-Asn-Ser-Lys-Phe-Ile-Gly-ILe-Thr-Glu-OH;
H-Gln-Tyr-Ile-Lys-Ala-Asn-Ser-Glu-Phe-Ile-Gly-ILe-Thr-Glu-OH;
H-Gln-Tyr-Ile-Glu-Ala-Asn-Ser-Lys-Phe-Ile-Gly-ILe-Thr-Glu-OH;
H-Gln-Tyr-Ile-Lys-Ala-Ala-Ser-Lys-Phe-Ile-Gly-ILe-Thr-Glu-OH.

3. Immunogenes Konjugat nach Anspruch 2, worin das Hapten von Viren oder Bakterien abgeleitet ist.

4. Immunogenes Konjugat nach Anspruch 3, worin das Hapten ein von Pneumococcen, Meningococcen, Haemophilus influenzae, Escherichia coli und hämolytischen Streptococcen abgeleitetes Polysaccharid ist.

5. Immunogenes Konjugat nach Anspruch 2, worin das Hapten ein synthetisches Peptid mit der Sequenz:

H-(Asn-Ala-Asn-Pro)$_n$-OH

ist, worin n einen Wert von 3 bis 40 aufweist.

6. Immunogenes Konjugat nach Anspruch 2, das sich zur Herstellung von synthetischen Vaccinen eignet, die zum Induzieren einer protektiven Antikörperantwort gegenüber einem interessierenden pathogenen Mittel befähigt sind, welches Konjugat nicht oder nur geringfügig genetisch beschränkt ist.

7. Immunogenes Konjugat nach Anspruch 5, das sich zur Herstellung einer Antimalariavaccine eignet, die zum Induzieren einer protektiven Antikörperantwort gegenüber durch Plasmodium falciparum verursachten Infektionen befähigt ist, welches Konjugat nicht oder nur geringfügig genetisch beschränkt ist.

8. Synthetische, zum Induzieren einer protektiven Antikörperantwort beim Menschen gegenüber einem pathogenen Mittel befähigte Vaccine, welche nicht oder nur geringfügig genetisch beschränkt ist, umfassend (a) eine immunologisch wirksame Menge des in Anspruch 2 beanspruchten Konjugats und (b) ein physiologisch annehmbares Medium, sowie gegebenenfalls (c) ein Hilfsmittel.

9. Synthetische, zum Induzieren einer protektiven Antikörperantwort beim Menschen gegenüber Plasmodium falciparum befähigten Antimalariavaccine, die nicht oder nur geringfügig genetisch beschränkt ist, umfassend eine immunologisch wirksame Menge des in Anspruch 5 beanspruchten Konjugats.

**Patentansprüche für folgende Vertragsstaaten : ES, GR**

1. Verfahren zur Herstellung von synthetischen Peptiden, die zum Induzieren der Proliferation von Human-Th-Lymphozyten in Assoziation mit verschiedenen Molekülen des humanen Histokompatibilitäts-Hauptkomplexes befähigt sind, mit der folgenden Aminosäuresequenz:

H-Gly-Glu-Ile-Gly-Asn-Asp-Pro-Asn-Arg-Asp-Ile-Leu-OH;

H-Gln-Tyr-Ile-Lys-Ala-Asn-Ser-Lys-Phe-Ile-Gly-Ile-Thr-Glu-OH;

H-Gln-Tyr-Ile-Lys-Ala-Asn-Ser-Lys-Phe-Ile-Gly-ILe-Thr-Glu-Leu-OH;

H-Lys-Ala-Asn-Ser-Lys-Phe-Ile-Gly-ILe-Thr-Glu-OH;

H-Gln-Tyr-Ile-Lys-Ala-Asn-Ser-Lys-Phe-Ile-Gly-ILe-Thr-OH;

H-Gln-Tyr-Ile-Lys-Ala-Asn-Ser-Lys-Phe-Ile-Gly-ILe-OH;

H-Gln-Ala-Ile-Lys-Ala-Asn-Ser-Lys-Phe-Ile-Gly-ILe-Thr-Glu-OH;

H-Gln-Phe-Ile-Lys-Ala-Asn-Ser-Lys-Phe-Ile-Gly-ILe-Thr-Glu-OH;

H-Gln-Tyr-Ile-Lys-Ala-Asn-Ser-Glu-Phe-Ile-Gly-ILe-Thr-Glu-OH;

H-Gln-Tyr-Ile-Glu-Ala-Asn-Ser-Lys-Phe-Ile-Gly-ILe-Thr-Glu-OH;

H-Gln-Tyr-Ile-Lys-Ala-Ala-Ser-Lys-Phe-Ile-Gly-ILe-Thr-Glu-OH,

dadurch gekennzeichnet, daß es die Stufen des Synthetisierens des angestrebten synthetischen

Peptids in fester Phase in einem automatischen Synthesizer in Anwesenheit eines handelsüblichen Polyacrylamidharzes durch Einführen der die erwartete Sequenz ausbildenden Aminosäuren, eine nach der anderen, durch eine Acylierungsreaktion, unter Verwendung der entsprechenden symmetrischen Anhydride oder eines entsprechenden Esters als aktive Form nach einem Schützen der N-endständigen Gruppe, soferne vorhanden, und der reaktiven funktionellen Gruppen in der Seitenkette, ein Abtrennen des solcherart gebildeten Peptids von dem Harz und ein Reinigen des Peptids umfaßt.

2. Verfahren zum Herstellung eines immunogenen Konjugats, dadurch gekennzeichnet, daß dieses Konjugat durch die Konjugation zwischen einem synthetischen Peptidträger mit einer der folgenden Aminosäuresequenzen:

H-Gly-Glu-Ile-Gly-Asn-Asp-Pro-Asn-Arg-Asp-Ile-Leu-OH;

H-Gln-Tyr-Ile-Lys-Ala-Asn-Ser-Lys-Phe-Ile-Gly-Ile-Thr-Glu-OH;

H-Gln-Tyr-Ile-Lys-Ala-Asn-Ser-Lys-Phe-Ile-Gly-ILe-Thr-Glu-Leu-OH;

H-Lys-Ala-Asn-Ser-Lys-Phe-Ile-Gly-ILe-Thr-Glu-OH;

H-Gln-Tyr-Ile-Lys-Ala-Asn-Ser-Lys-Phe-Ile-Gly-ILe-Thr-OH;

H-Gln-Tyr-Ile-Lys-Ala-Asn-Ser-Lys-Phe-Ile-Gly-ILe-OH;

H-Gln-Ala-Ile-Lys-Ala-Asn-Ser-Lys-Phe-Ile-Gly-ILe-Thr-Glu-OH;

H-Gln-Phe-Ile-Lys-Ala-Asn-Ser-Lys-Phe-Ile-Gly-ILe-Thr-Glu-OH;

H-Gln-Tyr-Ile-Lys-Ala-Asn-Ser-Glu-Phe-Ile-Gly-ILe-Thr-Glu-OH;

H-Gln-Tyr-Ile-Glu-Ala-Asn-Ser-Lys-Phe-Ile-Gly-ILe-Thr-Glu-OH;

H-Gln-Tyr-Ile-Lys-Ala-Ala-Ser-Lys-Phe-Ile-Gly-ILe-Thr-Glu-OH

und einem natürlichen oder synthetischen Peptid- oder Polysaccharidhapten, das von einem interessierenden pathogenen Mittel abgeleitet ist, hergestellt wird.

3. Verfahren nach Anspruch 2, worin das Hapten von Viren oder Bakterien abgeleitet ist.

4. Verfahren nach Anspruch 3, worin das Hapten ein von Pneumococcen, Meningococcen, Haemophilus influenzae, Escherichia coli und hämolytischen Streptococcen abgeleitetes Polysaccharidmolekül ist.

5. Verfahren nach Anspruch 2, worin das Hapten ein synthetisches Peptid mit der Sequenz:

H-(Asn-Ala-Asn-Pro)$_n$-OH

ist, worin n einen Wert von 3 bis 40 aufweist.

6. Verfahren zur Herstellung einer synthetischen Vaccine, die zum Induzieren einer protektiven Antikörperantwort beim Menschen gegenüber einem pathogenen Mittel befähigt ist, welche Vaccine nicht oder nur geringfügig genetisch beschränkt ist, gekennzeichnet durch Zusammenbringen von a) einer immunologisch wirksamen Menge des nach Anspruch 2 hergestellten Konjugats mit b) einem physiologisch annehmbaren Medium und gegebenenfalls c) eine Hilfsmittel.

7. Verfahren zur Herstellung einer synthetischen Antimalariavaccine, die zum Induzieren einer protektiven Antikörperantwort beim Menschen gegenüber Plasmodium falciparum befähigt ist, welche Vaccine nicht oder nur geringfügig genetisch beschränkt ist, umfassend eine immunologisch wirksame Menge des nach Anspruch 5 hergestellten Konjugats.

**Revendications**

**Revendications pour les Etats contractants suivants : AT, BE, CH, DE, FR, GB, LI, LU, NL, SE**

1. Peptides synthétiques capables de provoquer la prolifération de lymphocytes Th humains en association avec différentes molécules du complexe majeur d'histocompatibilité humain, et présentant l'une des séquences suivantes d'acides aminés :

   H-Gly-Glu-Ile-Gly-Asn-Asp-Pro-Asn-Arg-Asp-Ile-Leu-OH
   H-Gln-Tyr-Ile-Lys-Ala-Asn-Ser-Lys-Phe-Ile-Gly-Ile-Thr-Glu-OH
   H-Gln-Tyr-Ile-Lys-Ala-Asn-Ser-Lys-Phe-Ile-Gly-Ile-Thr-Glu-Leu-OH
   H-Lys-Ala-Asn-Ser-Lys-Phe-Ile-Gly-Ile-Thr-Glu-OH
   H-Gln-Tyr-Ile-Lys-Ala-Asn-Ser-Lys-Phe-Ile-Gly-Ile-Thr-OH
   H-Gln-Tyr-Ile-Lys-Ala-Asn-Ser-Lys-Phe-Ile-Gly-Ile-OH
   H-Gln-Ala-Ile-Lys-Ala-Asn-Ser-Lys-Phe-Ile-Gly-Ile-Thr-Glu-OH
   H-Gln-Phe-Ile-Lys-Ala-Asn-Ser-Lys-Phe-Ile-Gly-Ile-Thr-Glu-OH
   H-Gln-Tyr-Ile-Lys-Ala-Asn-Ser-Glu-Phe-Ile-Gly-Ile-Thr-Glu-OH
   H-Gln-Tyr-Ile-Glu-Ala-Asn-Ser-Lys-Phe-Ile-Gly-Ile-Thr-Glu-OH
   H-Gln-Tyr-Ile-Lys-Ala-Ala-Ser-Lys-Phe-Ile-Gly-Ile-Thr-Glu-OH

2. Conjugué immunogène constitué d'un peptide synthétique support lié par liaison covalente à un haptène, peptide synthétique ou naturel ou polysaccharide, dérivé d'un agent pathogène intéressant, dans lequel ledit peptide support présente l'une des séquences suivantes d'acides aminés :

   H-Gly-Glu-Ile-Gly-Asn-Asp-Pro-Asn-Arg-Asp-Ile-Leu-OH
   H-Gln-Tyr-Ile-Lys-Ala-Asn-Ser-Lys-Phe-Ile-Gly-Ile-Thr-Glu-OH
   H-Gln-Tyr-Ile-Lys-Ala-Asn-Ser-Lys-Phe-Ile-Gly-Ile-Thr-Glu-Leu-OH
   H-Lys-Ala-Asn-Ser-Lys-Phe-Ile-Gly-Ile-Thr-Glu-OH
   H-Gln-Tyr-Ile-Lys-Ala-Asn-Ser-Lys-Phe-Ile-Gly-Ile-Thr-OH
   H-Gln-Tyr-Ile-Lys-Ala-Asn-Ser-Lys-Phe-Ile-Gly-Ile-OH
   H-Gln-Ala-Ile-Lys-Ala-Asn-Ser-Lys-Phe-Ile-Gly-Ile-Thr-Glu-OH
   H-Gln-Phe-Ile-Lys-Ala-Asn-Ser-Lys-Phe-Ile-Gly-Ile-Thr-Glu-OH
   H-Gln-Tyr-Ile-Lys-Ala-Asn-Ser-Glu-Phe-Ile-Gly-Ile-Thr-Glu-OH
   H-Gln-Tyr-Ile-Glu-Ala-Asn-Ser-Lys-Phe-Ile-Gly-Ile-Thr-Glu-OH
   H-Gln-Tyr-Ile-Lys-Ala-Ala-Ser-Lys-Phe-Ile-Gly-Ile-Thr-Glu-OH

3. Conjugué immunogène conforme à la revendication 2, dans lequel l'haptène dérive de virus ou de bactéries.

4. Conjugué immunogène conforme à la revendication 3, dans lequel l'haptène est une molécule de polysaccharide dérivé de pneumocoques, de méningocoques, d'Haemophilus influenzae, d'Escherichia coli, ou de streptocoques hémolytiques.

5. Conjugué immunogène conforme à la revendication 2, dans lequel l'haptène est un peptide synthétique présentant la séquence :

H-(Asn-Ala-Asn-Pro)$_n$-OH

dans laquelle n vaut entre 3 et 40.

6. Conjugué immunogène conforme à la revendication 2, utile pour préparer des vaccins synthétiques capables de provoquer en réponse la production d'anticorps protecteurs contre un agent pathogène intéressant, réponse qui ne soit pas génétiquement limitée ou ne le soit que peu.

7. Conjugué immunogène conforme à la revendication 5, utile pour préparer un vaccin antimalarique capable de provoquer en réponse la production d'anticorps protecteurs contre des infections causées par Plasmodium falciparum, réponse qui ne soit pas génétiquement limitée ou ne le soit que peu.

8. Vaccin synthétique, capable de provoquer en réponse chez l'homme la production d'anticorps protecteurs contre un agent pathogène intéressant, réponse qui ne soit pas génétiquement limitée ou ne le soit que peu, et comprenant (a) une quantité immunologiquement efficace d'un conjugué conforme à la revendication 2 et (b) un milieu physiologiquement acceptable, ainsi qu'éventuellement (c) un adjuvant.

9. Vaccin antimalarique synthétique, capable de provoquer en réponse chez l'homme la production d'anticorps protecteurs contre des infections causées par Plasmodium falciparum, réponse qui ne soit pas génétiquement limitée ou ne le soit que peu, et comprenant une quantité immunologiquement efficace du conjugué conforme à la revendication 5.

**Revendications pour les Etats contractants suivants : ES, GR**

1. Procédé de préparation de peptides synthétiques capables de provoquer la prolifération de lymphocytes Th humains en association avec différentes molécules du complexe majeur d'histocompatibilité humain, et présentant l'une des séquences suivantes d'acides aminés :

H-Gly-Glu-Ile-Gly-Asn-Asp-Pro-Asn-Arg-Asp-Ile-Leu-OH
H-Gln-Tyr-Ile-Lys-Ala-Asn-Ser-Lys-Phe-Ile-Gly-Ile-Thr-Glu-OH
H-Gln-Tyr-Ile-Lys-Ala-Asn-Ser-Lys-Phe-Ile-Gly-Ile-Thr-Glu-Leu-OH
H-Lys-Ala-Asn-Ser-Lys-Phe-Ile-Gly-Ile-Thr-Glu-OH
H-Gln-Tyr-Ile-Lys-Ala-Asn-Ser-Lys-Phe-Ile-Gly-Ile-Thr-OH
H-Gln-Tyr-Ile-Lys-Ala-Asn-Ser-Lys-Phe-Ile-Gly-Ile-OH
H-Gln-Ala-Ile-Lys-Ala-Asn-Ser-Lys-Phe-Ile-Gly-Ile-Thr-Glu-OH
H-Gln-Phe-Ile-Lys-Ala-Asn-Ser-Lys-Phe-Ile-Gly-Ile-Thr-Glu-OH
H-Gln-Tyr-Ile-Lys-Ala-Asn-Ser-Glu-Phe-Ile-Gly-Ile-Thr-Glu-OH
H-Gln-Tyr-Ile-Glu-Ala-Asn-Ser-Lys-Phe-Ile-Gly-Ile-Thr-Glu-OH
H-Gln-Tyr-Ile-Lys-Ala-Ala-Ser-Lys-Phe-Ile-Gly-Ile-Thr-Glu-OH

procédé caractérisé en ce qu'il comporte les étapes consistant à synthétiser le peptide synthétique voulu en phase solide, dans un appareil de synthèse automatique, en présence d'une résine polyacrylamide du commerce, en introduisant un par un, par une réaction d'acylation, les acides aminés formant la séquence voulue, en utilisant comme forme active l'anhydride symétrique correspondant ou un ester correspondant, après avoir protégé, chaque fois qu'il y en a, le groupe N-terminal et les groupes fonctionnels réactifs situés dans la chaîne latérale, à séparer le peptide formé de la résine et à le purifier.

**2.** Procédé de préparation d'un conjugué immunogène, caractérisé en ce que l'on prépare ce conjugué par conjugaison entre un peptide synthétique support présentant l'une des séquences suivantes d'acides aminés :

H-Gly-Glu-Ile-Gly-Asn-Asp-Pro-Asn-Arg-Asp-Ile-Leu-OH
H-Gln-Tyr-Ile-Lys-Ala-Asn-Ser-Lys-Phe-Ile-Gly-Ile-Thr-Glu-OH

H-Gln-Tyr-Ile-Lys-Ala-Asn-Ser-Lys-Phe-Ile-Gly-Ile-Thr-Glu-Leu-OH
H-Lys-Ala-Asn-Ser-Lys-Phe-Ile-Gly-Ile-Thr-Glu-OH
H-Gln-Tyr-Ile-Lys-Ala-Asn-Ser-Lys-Phe-Ile-Gly-Ile-Thr-OH
H-Gln-Tyr-Ile-Lys-Ala-Asn-Ser-Lys-Phe-Ile-Gly-Ile-OH
H-Gln-Ala-Ile-Lys-Ala-Asn-Ser-Lys-Phe-Ile-Gly-Ile-Thr-Glu-OH
H-Gln-Phe-Ile-Lys-Ala-Asn-Ser-Lys-Phe-Ile-Gly-Ile-Thr-Glu-OH
H-Gln-Tyr-Ile-Lys-Ala-Asn-Ser-Glu-Phe-Ile-Gly-Ile-Thr-Glu-OH
H-Gln-Tyr-Ile-Glu-Ala-Asn-Ser-Lys-Phe-Ile-Gly-Ile-Thr-Glu-OH
H-Gln-Tyr-Ile-Lys-Ala-Ala-Ser-Lys-Phe-Ile-Gly-Ile-Thr-Glu-OH

et un haptène, peptide synthétique ou naturel ou polysaccharide, dérivé d'un agent pathogène intéressant.

**3.** Procédé conforme à la revendication 2, dans lequel l'haptène dérive de virus ou de bactéries.

**4.** Procédé conforme à la revendication 3, dans lequel l'haptène est une molécule de polysaccharide dérivé de pneumocoques, de méningocoques, d'Haemophilus influenzae, d'Escherichia coli, ou de streptocoques hémolytiques.

**5.** Procédé conforme à la revendication 2, dans lequel l'haptène est un peptide synthétique présentant la séquence :

H-(Asn-Ala-Asn-Pro)$_n$-OH

dans laquelle n vaut entre 3 et 40.

**6.** Procédé de préparation d'un vaccin synthétique, capable de provoquer en réponse chez l'homme la production d'anticorps protecteurs contre un agent pathogène intéressant, réponse qui ne soit pas génétiquement limitée ou ne le soit que peu, caractérisé en ce que l'on combine ensemble (a) une quantité immunologiquement efficace d'un conjugué préparé selon la revendication 2 et (b) un milieu physiologiquement acceptable, ainsi qu'éventuellement (c) un adjuvant.

**7.** Procédé de préparation d'un vaccin antimalarique synthétique, capable de provoquer en réponse chez l'homme la production d'anticorps protecteurs contre des infections causées par Plasmodium falciparum, réponse qui ne soit pas génétiquement limitée ou ne le soit que peu, et comprenant une quantité immunologiquement efficace du conjugué préparé selon la revendication 5.

Fig.1

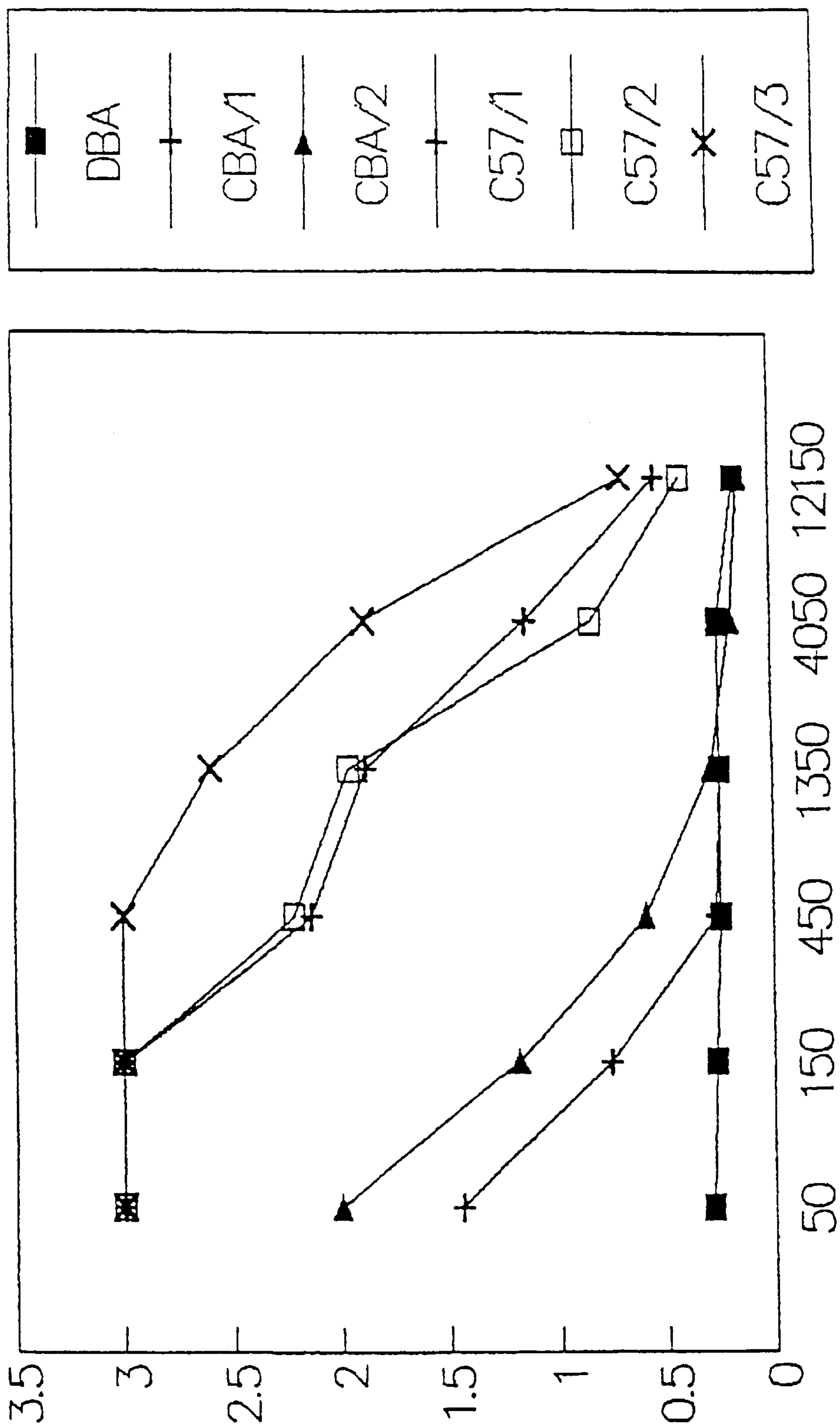
3.5
3
2.5
2
1.5
1
0.5
0
50
150
450
1350
4050
12150
DBA
CBA/1
CBA/2
C57/1
C57/2
C57/3

Fig.2

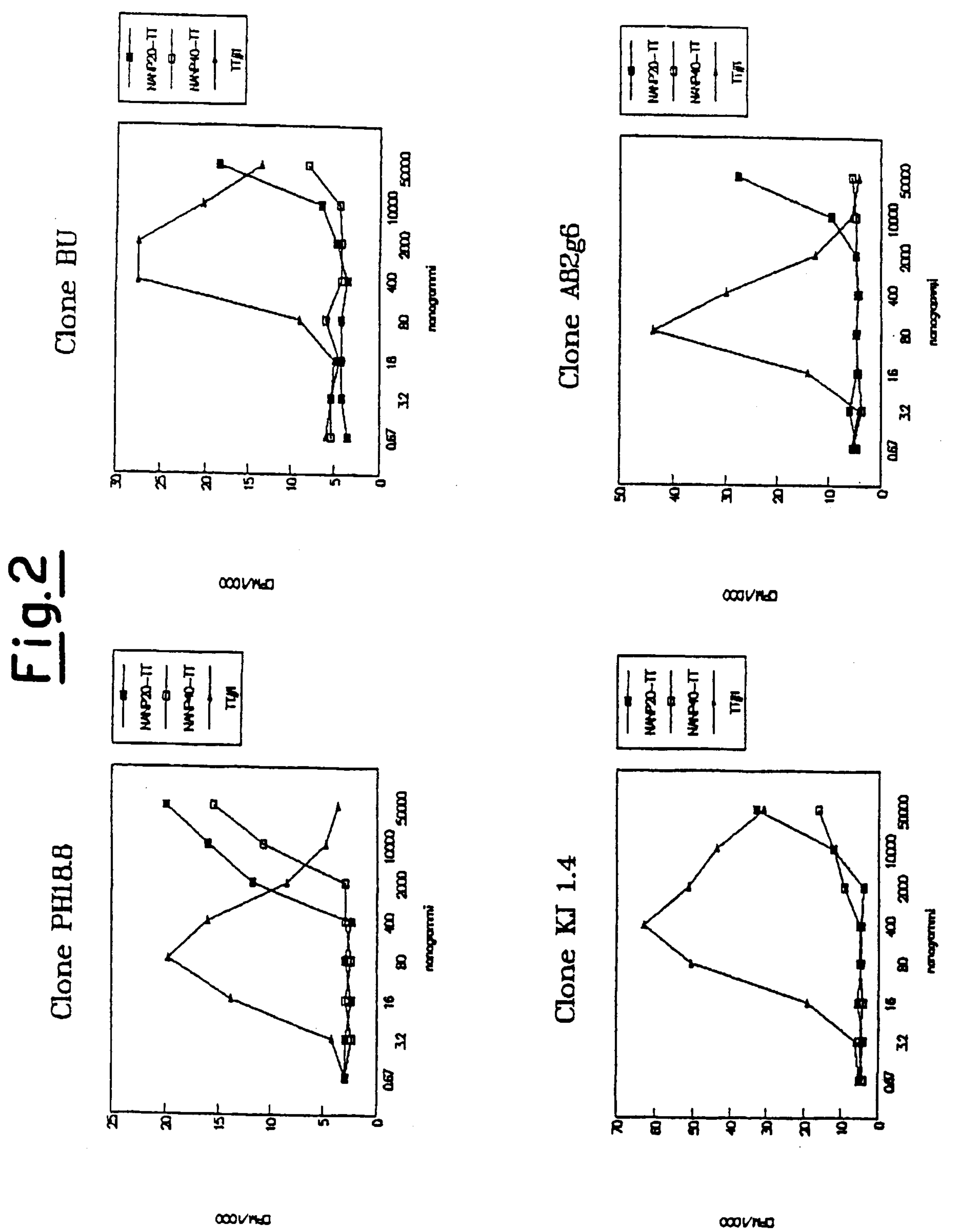
Clone BU
Clone AB2g6
Clone PH18.8
Clone KJ 1.4
NANP20-TT
NANP40-TT
nanogrammi